Europäisches Patentamt

European Patent Office

Office européen des brevets

(11) Veröffentlichungsnummer: **0 368 191**
**A1**

## (12) EUROPÄISCHE PATENTANMELDUNG

(21) Anmeldenummer: **89120472.9**

(22) Anmeldetag: **06.11.89**

(30) Priorität: **07.11.88 DE 8813905 U**

(43) Veröffentlichungstag der Anmeldung:
**16.05.90 Patentblatt 90/20**

(84) Benannte Vertragsstaaten:
**CH DE FR LI**

(51) Int. Cl.⁵: **A61M 5/24**

(71) Anmelder: **KIRCHNER & WILHELM GMBH & CO.**
**Eberhardstrasse 56**
**D-7144 Asperg 1(DE)**

(72) Erfinder: **Nothdurft, Klaus**
**Vaihinger Strasse 50**
**D-7000 Stuttgart 80(DE)**

(74) Vertreter: **Schmid, Berthold et al**
**Patentanwälte Dipl.-Ing. B. Schmid Dr. Ing. G.**
**Birn Falbenhennenstrasse 17**
**D-7000 Stuttgart 1(DE)**

(54) **Injektionsgerät.**

(57) Es sind bereits Injektionsgeräte mit in einer Kolbenführung (5) längsverschiebbarem Kolben (6) mit einstellbarem Arbeitshub (14) bekannt, bei denen mit der Kolbenführung (5) eine eine Kanülenhalterung (1) aufweisende Ampullenaufnahme (3) lösbar verbunden ist. Diese haben jedoch den Nachteil, daß die zu injizierende Menge immer neu eingestellt werden muß, wodurch die Gefahr von Fehleinstellungen besteht. Um nun eine Injektionsgerät zu schaffen, bei dem eine einmal vorgenommene Einstellung beibehalten wird, besteht der Kolben (6) aus zwei gegeneinander verschieb- und feststellbar verbundenen Längsteilen (8, 9), wobei es sich vorzugsweise um einen Hohlkolben (8) und eine in einem Gewinde (10) desselben gelagerte Kolbenstange (9) handelt.

Fig. 1

## Injektionsgerät

Die Erfindung bezieht sich auf ein Injektionsgerät mit in einer Kolbenführung längsverschiebarem Kolben mit einstellbarem Arbeitshub, wobei mit der Kolbenführung eine eine Kanülenhalterung aufweisende Ampullenaufnahme lösbar verbunden ist. Derartige Geräte werden beispielsweise von Diabetikern benutzt, welche sich regelmäßig eine bestimmte Menge des Ampulleninhalts injizieren müssen. Da Diabetiker vielfach sehbehindert sind, ist es bei den bekannten Geräten sehr nachteilig, daß die zu injizierende Menge immer neu eingestellt werden muß, wodurch die Gefahr von Fehleinstellungen besteht. Zwar können bei anderen bekannten Injektionsgeräten die zu injizierende Menge durch Klicken einer Rastung berechnet werden, jedoch kann es auch hier bei Rechenfehlern zu Falscheinstellungen kommen, die lebensgefährlich sein können.

Es ist die Aufgabe der Erfindung, ein Injektionsgerät der beschriebenen Art zu schaffen, bei dem eine einmal vorgenommene Einstellung beibehalten wird. Zur Lösung dieser Aufgabe besteht erfindungsgemäß der Kolben aus zwei gegeneinander verschieb- und feststellbar verbundenen Längsteilen. Dadurch kann die Länge des Kolbens und damit sein Arbeitshub verändert und festgestellt werden. Bei wiederholter Benutzung des Injektionsgeräts bleibt der einmal so eingestellte Arbeitshub des Kolbens immer der gleiche, wodurch immer die gleiche zu injizierende Menge aus der Ampulle herausgedrückt wird. Der Einstellungsfehler für die zu injizierende Menge hat sich somit erheblich reduziert.

Vorzugsweise sind als Längsteile ein in einem Hohlkolben gelagerte Kolbenstange vorgesehen, wobei zweckmäßig der Hohlkolben drehbar, aber unverschiebbar und die Kolbenstange verschiebbar, aber unverdrehbar in der Kolbenführung gelagert und durch Gewinde miteinander verbunden sind. Beim Drehen des Hohlkolbens verschiebt sich die in Drehrichtung festgelegte Kolbenstange in Längsrichtung des Injektionsgeräts. Damit ist eine einfache und sichere Möglichkeit zur Längenveränderung des Kolbens geschaffen. Die Längsführung läßt sich besonders einfach durch einen Stift der Kolbenstange, welcher in einen Längsschlitz der Kolbenführung eingreift, erreichen.

Zur erleichterten Einstellung des Arbeitshubes ist nach einem weiteren Merkmal der Erfindung an der Kolbenführung eine mit dem Hohlkolben unverdrehbar verbundene Einstellhülse gelagert. Dabei ist die Einstellhülse vorzugsweise mit der Kolbenführung durch ein Gewinde und mit dem Hohlkolben durch einen, in einen Längs schlitz eingreifenden Mitnehmer verbunden. Um die Längenänderung des Kolbens zu messen, weisen dabei der Längsschlitz eine Skala und der Mitnehmer einen Anzeigeknopf auf. Beim Drehen des Hohlkolbens mittels der Einstellhülse verschiebt sich diese und damit die Skala gegenüber dem Anzeigeknopf und zeigt so den eingestellten Arbeitshub des Kolbens an. Um auch eine hör- bzw. fühlbare Einstellung des Arbeitshubes zu erreichen, sind zweckmäßigerweise zwischen der Kolbenführung und dem Hohlkolben ein in Längsrichtung wirkendes, federbelastetes Rastglied und an einem der beiden Teile in Umfangsrichtung eine Rast vorgesehen. Bei jeder vollen Umdrehung der Einstellhülse spürt man das Ein- und Ausrasten und hört gleichzeitig ein leichtes Klickgeräusch. Die Gewinde zwischen Hohlkolben und Kolbenstange einerseits und Einstellhülse andererseits können verschiedene Steigungen aufweisen, wobei insbesondere die Steigung bei der Einstellhülse größer ist, um die Skala leichter ablesen zu können.

Weiterhin ist es von Vorteil, wenn die Ampullenaufnahme gegenüber der Kolbenführung längsverschiebbar und feststellbar gelagert ist. Damit wird beim wiederholten Injizieren einer bestimmten Menge aus einer einzigen Ampulle nach erfolgter Betätigung des Injektionsgeräts die Ampulle um den zuvor erfolgten Arbeitshub in Längsrichtung verstellt. Dabei ist es zweckmäßig, daß die Ampullenaufnahme auf ein Gewinde der Kolbenführung geschraubt ist.

Eine besonders vorteilhafte Weiterbildung der Erfindung sieht eine Spannvorrichtung für den Kolben vor. Bei bekannten Geräten erfolgt das Auslösen der zu injizierenden Menge über einen Druckknopf oder einen Drehknopf, welcher über die volle Länge des Arbeitshubs in Injektionsrichtung gedrückt bzw. gedreht werden muß. Dies ist nachteilig, da bei Diabetikern, welche die Injektionsgeräte selbst bedienen müssen, die Einstichstellen an teilweise schwer erreichbaren Orten des Körpers liegt und damit ein Verschwenken der Nadel im Körper beim Injizieren erfolgen kann. Bei einem Injektionsgerät mit der Spannvorrichtung braucht letztere zum Injizieren nur noch ausgelöst zu werden. Die Spannvorrichtung weist eine an der Kolbenführung längsverschiebbare, mit einer Spannstange versehene Spannhülse auf. Dabei ist zwischen einer Federauflage am freien Ende der Kolbenführung und einem Federteller der Spannstange eine Feder angeordnet und eine Rastvorrichtung zwischen Kolbenführung und Spannhülse vorgesehen. Die Rastvorrichtung besteht zweckmäßig aus einem mit der Spannstange verbundenen, mit Rastnase versehenen federbelasteten Rasthebel und die Spannhülse weist eine als Konus ausgebildete Auslösevorrichtung auf. Durch Verschieben der Spannhülse spannt die Spannstange die Feder, bis die Rastvorrichtung einrastet. Durch Zurückschieben der Spannhülse wird dann die Rastvorrichtung wieder ausgelöst, so daß die Spannstange auf den Hohlkolben auftrifft und diesen vorschnellen läßt.

Um einerseits die Kanüle bei Nichtbenutzung des Injektionsgerätes vor Beschädigungen zu schützen sowie Verletzungen zu vermeiden und andererseits den Einstich der Kanüle selbsttätig erfolgen zu lassen, ist die Kanülenhalterung gegen die Kraft einer Feder längsverschiebbar in einer die Länge der Kanüle überragenden Kanülenhülse gelagert. Im Ruhezustand wird die Kanüle durch die Feder in ihrer vollen Länge in die Hülse hineingedrückt. Sobald der Kolben gegen die Kanüle vorschnellt, wird auch die Kanülenhalterung nach vorne gedrückt, so daß die Kanüle austritt und der Injektionsvorgang beginnt.

Die Zeichnung zeigt Ausführungsbeispiele der Erfindung. Es stellen dar:

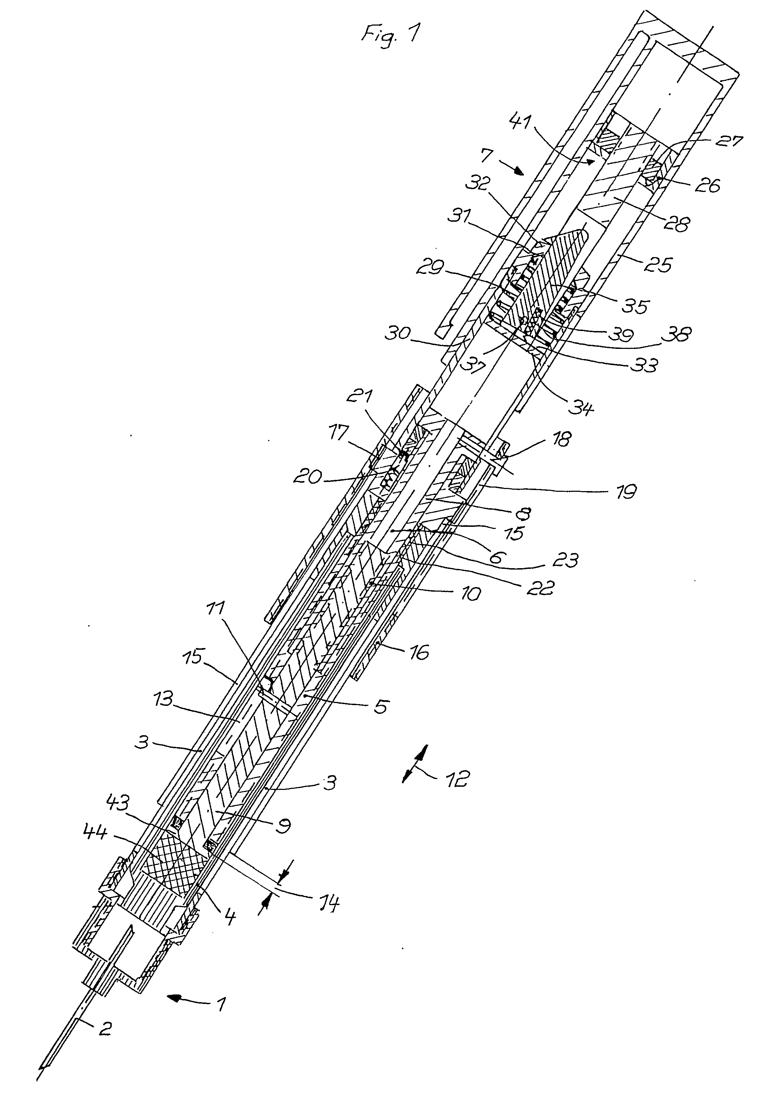

Fig. 1 einen Längsschnitt eines Injektionsgeräts im gespannten Zustand,

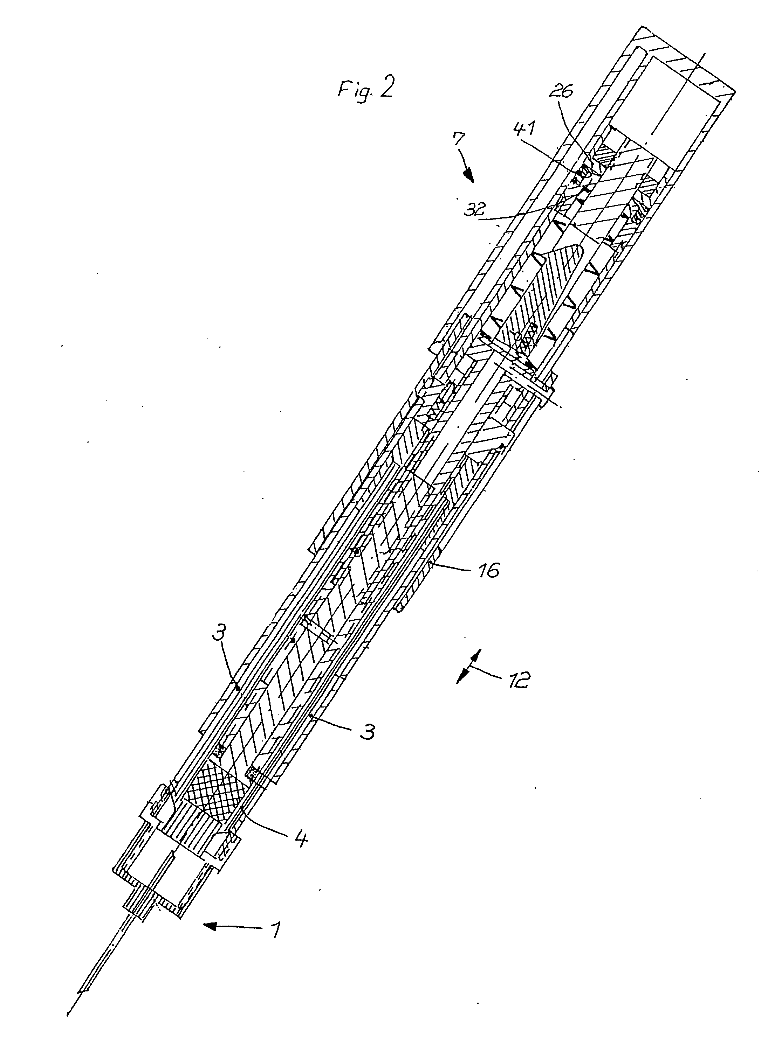

Fig. 2 eine Darstellung nach Fig. 1 im entspannten Zustand,

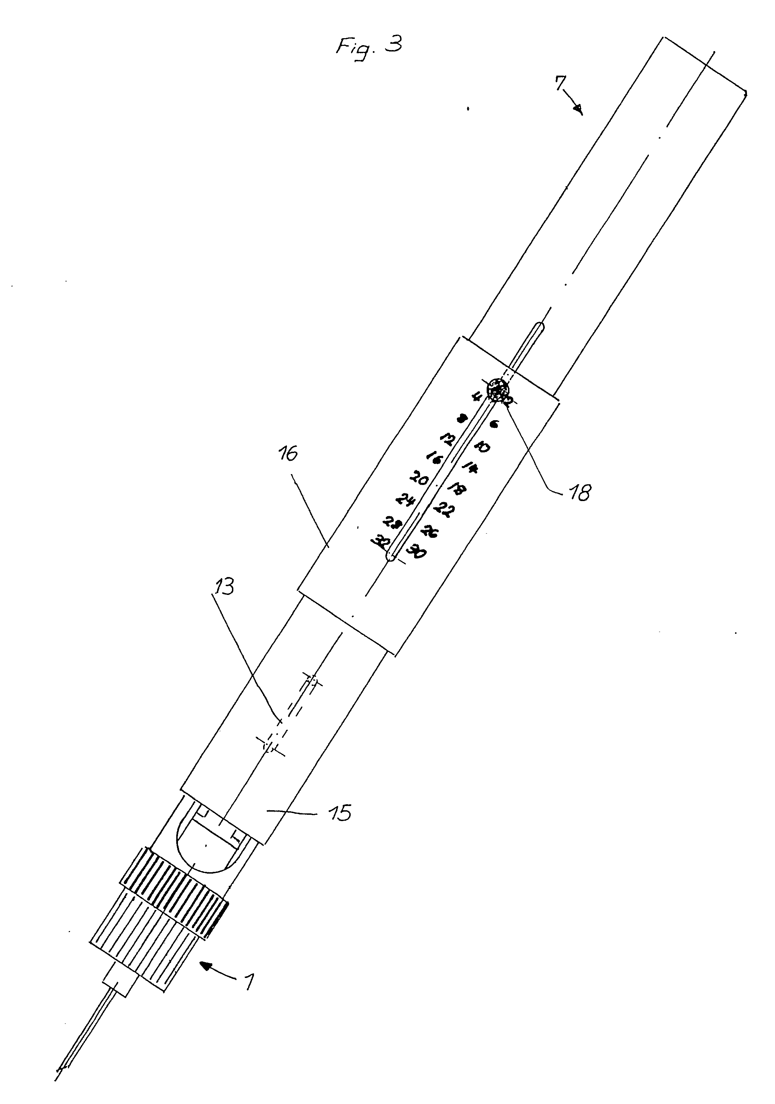

Fig. 3 eine Längsansicht des Injektionsgerätes im entspannten Zustand,

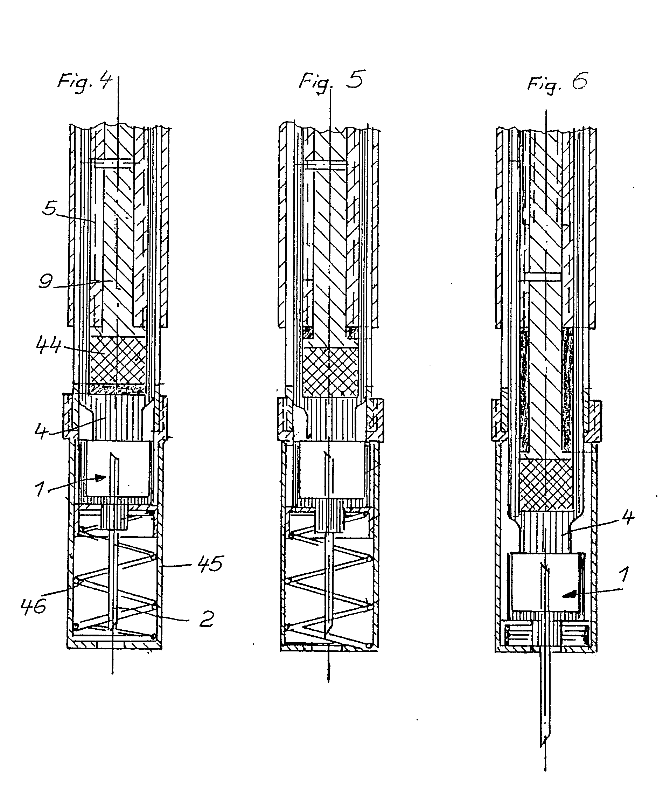

Fig. 4 einen abgebrochenen Längsschnitt einer weiteren Ausführungsform des Injektionsgerätes,

Fig. 5 eine Darstellung nach Fig. 4 mit um den Arbeitshub verschobener Kolbenstange,

Fig. 6 eine Darstellung nach Fig. 4 im entspannten Zustand.

Das Injektionsgerät weist eine Kanülenhalterung 1 mit Kanüle 2, eine damit verbundene Ampullenaufnahme 3 mit Ampulle 4, eine teilweise in die Ampulle 4 hineinragende Kolbenführung 5 für einen Kolben 6 und eine Spannvorrichtung 7 für den Kolben 6 auf. Der Kolben 6 besteht aus einem Hohlkolben 8 und einer Kolbenstange 9, welche durch ein Gewinde 10 verbunden sind. Ein Stift 11 ragt aus der Kolbenstange 9 quer zu deren Längsrichtung 12 heraus und greift in einen Längsschlitz 13 der Kolbenführung 5 ein. Dadurch wird bei Drehung des Hohlkolbens 8 die Kolbenstange 9 in Längsrichtung 12 verschoben, so daß die Länge des Kolbens und damit sein Arbeitshub 14 je nach Drehrichtung entweder vergrößert oder verkleinert wird.

Die Kolbenführung 5 sowie die Ampullenaufnahme 3 sind durch einen Außenmantel 15 abgedeckt. Die Kolbenführung 5 weist an ihrem oberen Ende ein Gewinde 17 auf, auf welchem sich eine Einstellhülse 16 befindet. Diese ist mit dem Hohlkolben 8 drehschlüssig durch einen in einen Längsschlitz 19 eingreifenden Mitnehmer 18 verbunden. Dabei befindet sich an der Einstellhülse 16 längs des Schlitzes 19 eine Skala und der Mitnehmer 18 ist als Anzeigeknopf ausgebildet, so daß seine Stellung an der Skala jeweils abgelesen werden kann. Beim Drehen der Einstellhülse 16 wird bei feststehendem Außenmantel 15 bzw. Kolbenführung 5 der Hohlkolben 8 mitgedreht. Gleichzeitig verschiebt sich einerseits die Einstell hülse 16 in Längsrichtung 12 und andererseits die Kolbenstange 9 in der gleichen Richtung. Das Gewinde 17 der Kolbenführung 5 ist mit einer größeren Steigung als das Gewinde 10 des Hohlkolbens 8 ausgestattet, so daß der so durch Drehung der Einstellhülse 16 relativ zur Kolbenführung 5 eingestellte Arbeitshub 14 in einem großen Maßstab auf der Skala ablesbar ist. Um einen einmal eingestellten Wert des Arbeitshubs 14 auch hörbar festzustellen, ist an der Kolbenführung 5 ein federbelastetes Rastglied 20 eingelassen, welches in eine am Hohlkolben 8 angeordnete Rastnut 21 bei jeder vollständigen Umdrehung der Einstellhülse 16 einrastet.

Um die Kolbenführung 5 in die Ampulle 4 unterschiedlich weit einzubringen, ist die Außenseite 22 der Kolbenführung 5 mit einem Gewinde 23 versehen, auf welches die Ampullenaufnahme 3 geschraubt ist, so daß durch Drehen der Ampullenaufnahme 3 diese gegenüber der Kolbenführung 5 in Längsrichtung 12 verschiebbar ist.

Die Spannvorrichtung 7 weist eine Spannhülse 25 mit einem Mitnehmeransatz 26 auf, welcher an einem Kopfteil 27 einer federbelasteten Spannstange 28 anliegt. Die Feder 29 der Spannstange 28 ist innerhalb einer gegenüber der Spannstange 28 feststehenden, mit der Kolbenführung 5 verbundenen Hülse 30 gelagert und liegt mit einem Ende 31 an einer Federauflage 32 der Hülsenwand und mit dem anderen Ende 33 am Federteller 34 der Spannstange 28 an. An der Spannstange 28 ist ein sich in Spannrichtung erstreckender federbelasteter Rasthebel 35 mit Rastnase 36 um seine Achse 37 schwenkbar angeordnet. Auf ihn wirkt eine Feder 38 ein, welche in eine Ausnehmung 39 des Rasthebels 35 eingreift und sich am Federteller 34 abstützt. Beim Bewegen der Spannhülse 25 in Spannrichtung 12 verschiebt der Mitnehmeransatz 26 die Spannstange 28, welche aus einer Öffnung der Hülse 30 herausragt, wodurch die Feder 29 zusammengedrückt wird. Der Rasthebel 35 bewegt sich ebenfalls in Spannrichtung, wobei anschließend seine Rastnase 36 mit dem Rand der Öffnung der Hülse 30 verrastet. Die Spannstange 28 befindet sich somit in einem verrasteten und die Feder 29 in gespanntem Zustand. Zum Lösen des Rasthebels 35 ist eine Auslösevorrichtung 41 vorgesehen, welche an der entgegen der Spannrichtung weisenden Seite des Mitnehmeransatzes 26 ausgebildet ist. Wird entgegen der Spannrichtung 12 auf die Spannhülse 25 gedrückt, so bewegt sich die Auslösevorrichtung 41 entlang der Spannstange 28, bis sie an der Rastnase 36 zur Anlage kommt und diese auslöst. Dadurch schnellt die Spannstange 28 in ihre entspannte Stellung, wobei sie auf den Hohlkolben 8 drückt und diesen um seinen eingestellten Arbeitshub in Injektionsrichtung bewegt. Das injektionsseitige Ende 43 der Kolbenstange 9 liegt an einem Propfen 44 an, welcher innerhalb der Ampulle 4 angeordnet ist und zur Abdichtung des Inhalts der Ampulle 4 dient. Der Propfen 44 wird ebenfalls um den Arbeitshub in Injektionsrichtung verschoben und drückt den Ampulleninhalt in die Kanüle 2.

Beim Benutzen des Injektionsgeräts schraubt man zuerst die Kanülenaufnahme 1 von der Ampullenaufnahme 3 ab und setzt die Ampulle 4 in die Ampullenaufnahme 3 ein. Anschließend stellt man durch Verdrehen der Einstellhülse 16 gegenüber dem Außenmantel 15 die gewünschte zu injizierende Menge ein, wobei sich der Anzeigeknopf 18 relativ zur Einstellhülse 16 bewegt und so die jeweils eingestellte Menge und damit den Arbeitshub des Kolbens 6 markiert.

Sobald die richtige Einstellung erreicht ist, wird die Ampullenaufnahme 3 in Spannrichtung auf die Kolbenführung 5 aufgeschraubt, bis der am unteren Ende 43 der Kolbenstange 9 anliegende Propfen 44 in Berührung mit dem Ampulleninhalt kommt. Beim weiteren Aufschrauben der Ampullenaufnahme verschiebt nun der gegen den Ampulleninhalt sich abstützende Propfen 44 den Kolben 6 in Spannrichtung, bis das Ende 43 der Kolbenstange 9 zur Anlage mit dem kanülenseitigen Ende der Kolbenführung 5 kommt und ein Verschieben der Kolbenstange 9 nicht weiter möglich ist. Bei einem kurzen weiteren Einschrauben der Ampullenaufnahme 3 wird der Ampulleninhalt durch die Kanüle 2 herausgedrückt, so daß diese luftleer ist. Die Spannvorrichtung 7 wird durch Ziehen der Spannhülse 25 in Spannrichtung 12 in ihre wirksame Stellung gebracht. Das Injektionsgerät ist nun einsatzbereit. Es wird in den Körper des Patienten eingestochen und die Spannvorrichtung 7 wird ausgelöst, wodurch der Kolben 6 um den eingestellten Arbeitshub in Injektionsrichtung bewegt wird und über den Propfen 44 die eingestellte Menge des Ampulleninhalts in die Kanüle 2 drückt. Um das Injetionsgerät wieder für die gleiche Menge des zu injizie renden Ampulleninhalts bereit zu machen, wird die Ampullenaufnahme 3 um den Arbeitshub 14 auf die Kolbenführung 5 aufgeschraubt, bis wiederum das Ende 43 der Kolbenstange 9 zur Anlage mit der Kolbenführung 5 kommt. Nach dem Spannen kann die nächste Injektion erfolgten. Beim wiederholten Injizieren gleicher Mengen des Ampulleninhalts genügt bei dem Injektionsgerät eine einmalige Einstellung der zu injizierenden Menge an der Einstellhülse 16.

Die Fign. 6 bis 8 zeigen eine Variante der Erfindung, wobei das Injektionsgerät als Automat arbeitet. Dabei ist die Kanülenhalterung 1 gegen die Kraft einer Feder 46 längsverschiebbar in einem Kanülengehäuse 45 gelagert, wobei die Länge des Kanülengehäuses 45 etwas größer als diejenige der Kanüle 2 ist. Infolgedessen ist die Kanüle 2 in gespanntem Zustand des Gerätes in der Kanülenhülse 45 versenkt. Beim Auslösen der Spannvorrichtung 7 wird zuerst die verschiebbare federbelastete Kanülenhalterung 1 beim Auftreffen des Propfens 44 auf den Ampulleninhalt durch dessen Widerstandskraft in den Körper des Patienten gestochen. Nach diesem schnellen Einstichvorgang erfolgt das langsame, vom Innendurchmesser der Kanüle 2 abhängige Ausdrücken der eingestellten Menge des Ampulleninhalts.

## Ansprüche

1 . Injektionsgerät mit in einer Kolbenführung (5) längsverschiebbarem Kolben (6) mit einstellbarem Arbeitshub (14), wobei mit der Kolbenführung (5) eine eine Kanülenhalterung (1) aufweisende Ampullenaufnahme (3) lösbar verbunden ist, dadurch gekennzeichnet, daß der Kolben (6) aus zwei gegeneinander verschieb-und festellbar verbundenen Längsteilen (8, 9) besteht.

2. Gerät nach Anspruch 1, dadurch gekennzeichnet, daß als Längsteile eine in einem Hohlkolben (8) gelagerte Kolbenstange (9) vorgesehen sind.

3. Gerät nach Anspruch 2, dadurch gekennzeichnet, daß der Hohlkolben (8) dreh- aber unverschiebbar und die Kolbenstange (9) verschiebaber unverdrehbar in der Kolbenführung (5) gelagert und durch Gewinde (10) miteinander verbunden sind.

4. Gerät nach einem oder beiden der vorhergehenden Ansprüche 2 oder 3, dadurch gekennzeichnet, daß die Kolbenstange (9) einen in einen Längsschlitz (13) der Kolbenführung (5) eingreifenden Stift (11) aufweist.

5. Gerät nach einem oder mehreren der vorhergehenden Ansprüche 2 bis 4, dadurch gekennzeichnet, daß an der Kolbenführung (5) eine mit dem Hohlkolben (8) unverdrehbar verbundene Einstellhülse (6) drehbar gelagert ist.

6. Gerät nach Anspruch 5, dadurch gekennzeichnet, daß die Einstellhülse (16) mit der Kolbenführung (5) durch ein Gewinde (17) und mit dem Hohlkolben (8) durch einen in einen Längsschlitz (19) eingreifenden Mitnehmer (18) verbunden ist, wobei insbesondere der Längsschlitz (19) eine Skala und der Mitnehmer (18) einen Anzeigeknopf aufweisen.

7. Gerät nach einem oder mehreren der vorhergehenden Ansprüche 2 bis 6, dadurch gekennzeichnet, daß zwischen der Kolbenführung (5) und dem Hohlkolben (8) ein in Längsrichtung wirkendes, federbelastetes Rastglied (20) und an einem der beiden Teile (5, 8) in Umfangsrichtung eine Rast (21) vorgesehen sind.

8. Gerät nach den Ansprüchen 3 und 6, dadurch gekennzeichnet, daß die Gewinde (10, 17) zwischen dem Hohlkolben (8) und der Kolbenstange (9) einerseits und der Einstellhülse (16) andererseits verschiedene Steigungen aufweisen.

9. Gerät nach einem oder mehreren der vorhergehenden Ansprüche, dadurch gekennzeichnet, daß die Ampullenaufnahme (3) gegenüber der Kolbenführung (5) längsverschieb- und feststellbar gelagert ist.

10. Gerät nach Anspruch 9, dadurch gekennzeichnet, daß die Ampullenaufnahme (3) und die Kolbenführung (5) durch ein Gewinde (23) verbunden sind.

11. Gerät nach einem oder mehreren der vorhergehenden Ansprüche, dadurch gekennzeichnet, daß die Kolbenführung (5) längsverschiebbar eine mit einer begrenzt verschiebbaren Spannstange (28) versehene Spannhülse (25) aufweist, wobei zwischen einer Federauflage (32) am freien Ende der Kolbenführung (5) und einem Federteller (34) der Spannstange (28) eine Feder (29) angeordnet und eine Rastvorrichtung (35, 36) zwischen Kolbenführung (5) und Spannhülse (25) vorgesehen sind.

12. Gerät nach Anspruch 11, dadurch gekennzeichnet, daß die Rastvorrichtung einen mit der Spannstange (28) verbundenen, mit Rastnase (36) versehenen, federbelasteten Rasthebel (35) und die Spannhülse (25) einen als Konus ausgebildeten Auslösevorrichtung (41) aufweisen.

13. Gerät nach einem oder mehreren der vorhergehenden Ansprüche, dadurch gekennzeichnet, daß die Kanülenhalterung (1) gegen die Kraft einer Feder (46) längsverschiebbar in einer die Länge der Kanüle (2) überragenden Kanülenhülse (45) gelagert ist.

Fig. 1

EP 0 368 191 A1

Fig. 2

Fig. 3

Fig. 4   Fig. 5   Fig. 6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 268 191 (WILHELM HASELMEIER) <br> * Ansprüche 1-14; Abbildungen 1-6 * | 1-8 | A 61 M 5/24 |
| A | | 9-10 | |
| | --- | | |
| A | US-A-4 592 745 (REX et al.) <br> * Abbildungen 2-3,10 * | 11-12 | |
| | --- | | |
| A | US-A-3 046 985 (CANDELARIO SAENZ) <br> * Abbildungen 2-3 * | 13 | |
| | ----- | | |

RECHERCHIERTE SACHGEBIETE (Int. Cl.5)

A 61 M

Der vorliegende Recherchenbericht wurde für alle Patentansprüche erstellt

| Recherchenort | Abschlußdatum der Recherche | Prüfer |
|---|---|---|
| DEN HAAG | 09-02-1990 | MIR Y GUILLEN V. |